# EUROPEAN PATENT APPLICATION

(11) **EP 2 471 528 A1**
(43) Date of publication of application: **04.07.2012**
(21) Application number: 10197296.6
(22) Date of filing: 29.12.2010
(51) Int. Cl.: A61K 31/23, A61K 9/00

(54) **A preparation method for suspension of cetyl myristate and/or cetyl palmitate**

(71) Applicant: Deva Holding Anonim Sirketi, Kucukcekmece/Istanbul (TR)
(72) Inventor: HAAS, Philipp Daniel, 34303, ISTANBUL (TR); FIRAT, Omer Faruk, 34303, ISTANBUL (TR); KANDEMIR, Levent, 34303, ISTANBUL (TR); KOC, Fikret, 34303, ISTANBUL (TR); SIVASLIGIL, Ramazan, 34303, ISTANBUL (TR)

(57) **Abstract**

This invention is related to a preparing method for pharmaceutical and/or dietary supplement suspension formulations of cetyl myristate or cetyl palmitate or combination of cetyl myristate and cetyl palmitate.

## Description

### Technical Field

This invention is related to a preparing method for pharmaceutical and/or dietary supplement suspension formulations of cetylated fatty acids, especially cetyl myristate or cetyl palmitate or combination of cetyl myristate and cetyl palmitate.

### Background Art

Cetyl palmitate is derived from the fatty acid, palmitic acid which occurs as the glycerol ester in many oils and fats such as palm oil or Chinese vegetable tallow. A synthetic method of preparation is to react palmitoyl chloride and cetyl alcohol in the presence of magnesium. See the Merck Index, 12th edition at page 336. Reference is also made to US US3169099 A (SOCONY MOBIL OIL CO INC) 02.09.1965 patent which discloses a biosynthetic method of producing cetyl palmitate.

US 4,113,881 A (DIEHL HARRY WELDON) 12.09.1978 discloses that the administration of an effective amount of cetyl myristoleate to a mammal is useful in inhibiting or relieving the symptoms of inflammatory rheumatoid arthritis in mammals.

US 5569676 A (DIEHL, HARRY W ) 29.10.1996 US 5,569,676 discloses the use of cetyl myristoleate in the treatment of osteo-arthritis.

WO 01/85162 A (MERACOL CORP LTD ET.AL.) 15.11.2001 discloses the use of cetyl myristate and/or cetyl palmitate in the treatment of irritable bowel syndrome or disease. Patent embraces that cetyl myristate comprises 50-98 wt.% of the mixture, preferably, the myristate and palmitate are in a weight ratio of 95:5. The oral dosage unit is a capsule and contains 5-400 mg of the cetyl myristate or the mixture of the cetyl myristate and the cetyl palmitate. It also includes an excipient and/or diluent, preferably silicon dioxide, calcium phosphate and/or magnesium oxide. Preferably said mixture is in a capsule and it includes a pharmaceutically acceptable excipient and/or diluents. Preferably the dosage unit includes silicon dioxide, calcium phosphate and/or magnesium oxide. Liquid formulation, where an amount of liquid equivalent to at least 4 capsules is prescribed which is to be taken 3 times daily. That is 4200 mg of cetyl myristate or the mixture of cetyl myristate and cetyl palmitate. That mixture comprises by weight 95% cetyl myristate and 5% cetyl palmitate by weight. In addition, added excipients were present in the non gelatin two part capsule case.

WO 01/85163 A (MERACOL CORP LTD ET.AL.) 15.11.2001 discloses the use of cetyl myristate and/or cetyl palmitate in the treatment of eczema and/or psoriasis. Accordingly, capsule also includes a pharmaceutically acceptable excipient and/or diluent. These are silicon dioxide, calcium phosphate and/or magnesium oxide. The dosage unit can also be a wax-like solid or can be an orally consumable liquid composition (eg; made up with a general pharmacy type carrier such as methyl cellulose).

WO 2005/118070 A (MERACOL CORP LTD ET.AL.) 15.12.2005 discloses the treatment of multiple sclerosis with the use of cetyl myristate and/or cetyl palmitate.The cetyl myristate; or combination of cetyl myristate and cetyl palmitate is administered simultaneously, separately or sequentially.

WO 03/018731 A (MERACOL CORP LTD) 06.03.2003 defines the process preparing a mixture of cetyl myristate (50-98 wt.%) and cetyl palmitate, for use in the formulation of cosmetics and pharmaceuticals.

WO 03/045374 A (MERACOL CORP LTD ET.AL.) 05.06.2003 discloses the use of cetyl myristate and/or cetyl palmitate in a method of treatment and/or prophylaxis of a mammal for at least the symptoms of treating asthma, chronic obstructive pulmonary disease and/or other respiratory difficulties.

WO 03/026640 A (MERACOL CORP LTD ET.AL.) 03.04.2003 discloses the use of cetyl myristate and/or cetyl palmitate in the treatment of food allergies and/or food intolerances.

WO 01/85164 A (MERACOL CORP LTD ET.AL.) 15.11.2001 discloses the use of cetyl myristate and/or cetyl palmitate in the treatment of herpes.

### Summary of invention

This invention discloses a preparation method for suspension formulations of cetylated fatty acids, especially cetyl myristate or cetyl palmitate or combination of cetyl palmitate and cetyl myristate.

### Technical Problem

Cetyl myristate or cetyl palmitate or combination of cetyl myristate and cetyl palmitate can be used as active pharmaceutical ingredients (API) in pharmaceutical or dietary supplement formulations in addition to their excipient properties. However, preparing of suspension of those is very hard, since at preparation stage particles of cetyl myristate and cetyl palmitate are agglomerated when contact with liquid and de-agglomeration becomes problematic. Therefore, in the course of preparation of suspension, agglomeration problem should be eliminated.

### Solution to Problem

It is invented that agglomeration, which is occurred, in the course of preparation can be removed by warming of preparation liquid to equal or exceeding 40°C and using of homogenizator simultaneously with cooling.

### Description of embodiments

According to this invention, it is invented that suspension of cetyl myristate or cetyl palmitate or combination of cetyl myristate and cetyl palmitate is properly prepared where preparation liquid, which is based on suspension, is warmed to temperatures equal or exceeding 40°C. Less than 40°C, to be dispersed in preparation liquid is so difficult and particles of cetyl myristate or cetyl palmitate or combination of cetyl myristate and cetyl palmitate are agglomerated.

Cetyl myristate or cetyl palmitate or combination of cetyl myristate and cetyl palmitate may be warmed in preparation liquid with or without excipient or mixtures of excipients or they may be loaded to pre-warmed liquid.

Warming may be performed inside or outside of homogenizator.

The term of "preparation liquid" means that any kind of mixture or suspensions before bottling.

Another problem is additionally faced at cooling stage. If preparation liquid warmed over 40°C is leaved to be cooled,dispersed particles are again agglomerated and become rotund form with cooling. To solve this problem, it is invented that preparation liquid,which is based on suspension, is passed through a homogenizator or treated with a homogenizator contemporaneously with cooling, namely during the cooling suspension is passed through homogenizator or treated with a homogenizator.

Cooling may be performed to room or ambient temperatures.

The term of homogenizator is not limited herein and covers all kind of apparatuses, whatsoever terminology, which have same or similar function or functions with homogenizator. High-speed mixers, blenders, colloid mills, homogenizers, dispensers, ultrasonic devices and the like may be used.

In suspension formulations of cetyl myristate or cetyl palmitate or combination of cetyl myristate and cetyl palmitate, excipient or mixtures of excipients can be used such as viscosity enhancing agent, preservative, flavor, flavour enhancer agent, antifoaming agent, pH adjuster, mixtures thereof and the like.

### Example

In accordance with this invention a suspension is prepared as following steps:

a) Sweetener or mixtures of sweeteners and preservative or mixtures of preservatives and flavor enhancer or mixtures of flavor enhancers are mixed with water.

b) Flavor or mixtures of flavors and antifoaming agent or mixtures of antifoaming agents are mixed with the mixture obtained from step a

c) The obtained mixture from step b and cetyl palmitate and cetyl myristate are mixed for preferably 30-35 minutes and warmed to equal or exceeding 40°C, preferably 55°C

d) The obtained mixture from step c is treated with homogenizator during the cooling to room or ambient temperatures, preferably 30-35°C.

e) Viscosity enhancer or mixtures of viscosity enhancers are added to the mixture obtained from step d

f) pH adjuster or mixtures of pH adjusters are added to the mixture obtained from step e.

g) The mixtures obtained from step g is the final suspension and it is bottled.

## Claims

1. A preparation method for suspension of cetyl myristate or cetyl palmitate or combination of cetyl myristate and cetyl palmitate **characterized in that** preparation liquid is warmed to temperature equal or exceeding 40°C and said liquid is passed through a homogenizator or treated with a homogenizator during the cooling phase.

2. According to claim 1, cetyl myristate or cetyl palmitate or combination of cetyl myristate and cetyl palmitate are presence in preparation liquid and warmed altogether.

3. According to claim 1, cetyl myristate or cetyl palmitate or combination of cetyl myristate and cetyl palmitate are added to pre-warmed preparation liquid.

4. According to preceding claims, preparation liquid is preferably warmed to 55°C.

5. According to claim 1 preparation liquid is cooled to room or ambient temperatures.

6. According to claim 5, preparation liquid is preferably cooled to 30-35°C.

7. According to claim 1 preparation liquid contains cetyl myristate or cetyl palmitate or combination of cetyl myristate and cetyl palmitate and viscosity enhancing agent and preservative and flavour and flavour enhancer agent and antifoaming agent and pH adjuster and water.
